# EUROPEAN PATENT SPECIFICATION

(11) **EP 0 662 466 B1**
(45) Date of publication and mention of the grant of the patent: **19.03.1997**
(21) Application number: 94120067.7
(22) Date of filing: 19.12.1994
(51) Int. Cl.: C07C 51/09, C07C 67/03, C07C 69/82, C08J 11/24, C07C 63/26

(54) **Continuous process for the recovery of terephthalic acid from waste or used products of polyalkylene terephthalate polymers**
Kontinuierliches Verfahren zur Gewinnung von Terephthalsäure aus Abfällen oder gebrauchten Produkten aus Polyalkylenterephthalat Polymeren
Procédé continu de récupération de l'acide téréphtalique à partir de déchets ou de produits utilisés de polyalkylène téréphtalate polymères

(30) Priority: 11.01.1994 IT MI940017
(43) Date of publication of application: 12.07.1995
(73) Proprietor: MONTEFIBRE S.p.A., I-20124 Milano (IT)
(72) Inventor: Socrate, Contessa, Salerno (IT); Vosa, Renato, Casagione, (Caserta) (IT)
(74) Representative: Fusina, Gerolamo

(56) References cited:
- EP-A- 0 484 963
- EP-A- 0 599 309
- IT-A- 729 187
- US-A- 4 578 502

## Description

The present invention relates to a process for the recovery of waste and used products of polyalkylene terephthalate.

More specifically, the present invention relates to a process for the recovery of terephthalic acid and alkylene glycols from waste and used products of polyalkylene terephthalate.

Polyalkylene terephthalates are polymeric products with a high molecular weight which are well-known in the art. They are obtained by the polytransesterification of terephthalic acid or one of its alkyl-esters with an aliphatic glycol. In the specific case of polyethylene terephthalate, which is the most common polyester on the market, this is generally produced by reacting terephthalic acid or one of its alkyl esters, such as dimethyl terephthalate, with ethylene glycol, in the presence of a catalyst, to form the dihydroxyethyl-terephthalate monomer which is subsequently polymerized to a high molecular weight using the known polymerization techniques by condensation.

Polyalkylene terephthalates with a high molecular weight, and in particular polyethylene terephthalate, are industrially applied in the production of films for packaging, fibres, electric insulators, moulded articles, food containers, such as bottles, etc. The various operating or production processes generate, however, a considerable quantity of waste or by-products, such as shavings, polymer cuttings, thread and textile waste, etc., which negatively affect the cost of the finished product. In addition, the mass of these products, is disposed after use in rubbish dumps. These waste or used products consequently create enormous problems both for the environment and for their disposal.

Various processes have been proposed for recycling these waste products, mainly based on the recovery of their constituents and in particular the most costly component i.e. terephthalic acid. The various known processes have numerous disadvantages however due to the fact that the regenerated products contain impurities which are difficult to separate and which jeopardize their subsequent re-use.

One method proposed consists in dissolving the waste products in dihydroxyethyl terephthalate monomer in the molten state and recycling the solution obtained in polycondensation. The polyester thus obtained has lower properties than those of polyester obtained from vergin monomers.

Another method proposed consists in degrading the polymer with an alkyl alcohol with a low molecular weight and recycling the degraded product. For example, patent US-A-3.257.335 describes a degradation process of waste products of polyalkylene terephthalate by treatment with ethylene glycol. The process consists in dissolving and reacting the waste products of polyalkylene terephthalate with ethylene glycol to prepare di-hydroxyethyl terephthalate monomer and oligomers with a low molecular weight (dimers, trimers etc.), which are subsequently mixed with the vergin monomer and then polymerized. The polymer obtained with this process has limited properties, especially when a high ratio of recovery products is used. This deterioration of the properties also seems to be due to the formation of diethylene glycol during the reaction with ethylene glycol.

As an alternative, patents US-A-2.884.443, US-A-3.488.298 and US-A-3.776.945 propose degradation of the polyalkylene terephthalate with methanol to form dimethyl terephthalate. The dimethyl terephthalate recovered is then added in the transesterification phase in the production process of polyalkylene terephthalate. The main disadvantage of this process is the difficulty of introducing the solid waste products of polyester at the high pressure required to obtain the degradation of the polymer. For this reason the process is carried out in batch with relative problems of storage and all the drawbacks associated with a batch process.

A proposal has also been made to carry out the degradation of the waste products of polyalkylene terephthalate with a two-step process. In the first step the waste products are treated with ethylene glycol to prepare a solution containing dihydroxyethylene terephthalate and oligomers with a low molecular weight and in the second step the solution is treated with methanol to further degrade the oligomers and convert the terephthalates to dimethyl terephthalate. A similar process using an exchange catalyst is disclosed in patent DDR-A-68.500. This process however, also has the above disadvantages, as impurities such as diethylene glycol and comonomers and/or additives commonly used in the production of polyester, are entrapped in the dimethyl terephthalate obtained by crystallization of the mother liquor and their elimination by subsequent crystallization is a technically complex and costly operation.

European patent EP-A-0.484.963 describes a continuous process for the preparation of dimethyl terephthalate from the waste products of polyalkylene terephthalate by treatment with methanol vapours at a temperature higher than 230°C, continuous removal of methanol vapours, dimethyl terephthalate and olefinic glycol from the reaction zone, separation of the methanol and recovery of the dimethyl terephthalate.

Patent US-A-5.015.528 discloses a continuous process for the recovery of ethylene glycol and dimethyl terephthalate from polyethylene terephthalate consisting in passing superheated methanol vapours through a solution obtained by dissolving polymer waste products in an oligomer of ethylene glycol and terephthalic acid or its methyl ester and recovering the ethylene glycol and dimethyl terephthalate by distillation. These continuous processes enable the production of dimethyl terephthalate and ethylene glycol without inorganic residues and heteropolymers, enabling them to be more easily purified but without solving the problem of the separation of the comonomers. These processes, however, have the serious technical problem of having to be carried out extremely close to where the dimethyl terephthalte is used. It is, in fact, known that dimethyl terephthalate is not very stable and there are consequently technical problems for its transport.

Processes have also been proposed for the recovery of waste products of polyalkylene terephthalate by both continuous and batch hydrolysis to obtain pure terephthalic acid directly by crystallization. Hydrolysis processes are described in patents US-A-3.321.510, US-A-4.605.762 and GB-A-2.123.403. Not even these processes, however, solve the problem of the impurities due to both the formation of hydroyethyl-methyl terephthalate and to the presence of comonomers of the starting polyester. In addition, the hydrolysis process requires the use of waste products which are absolutely without pigments, heteropolymers or various additives as well as extraneous materials such as paper or aluminium films, and they must consequently by subjected to a preliminary purification process which considerably influences the cost of the products obtained.

The present invention provides a continuous process for the recovery of terephthalic acid and alkylene glycol from the waste or used products of the production and transformation of polyester which does not have the above disadvantages.

More specifically, the present invention provides a continuous process for the degradation of polyalkylene terephthalate to obtain "fibre grade" terephthalic acid i.e. without impurities which can jeopardize its re-use in the production of polyesters for fibres.

In its more general aspect, the process of the present invention consists in subjecting the waste or used products of polyalkylene terephthalate to two consecutive treatments, the first carried out with superheated methanol vapours (methanolysis) and the second with water or water vapour at a high temperature (hydrolysis).

The present invention consequently relates to a continuous process for the recovery of terephthalic acid from the waste or used products of polyalkylene terephthalate polymers which comprises in:
(a) treating said polymer waste products with methanol vapours, at atmospheric pressure and at a temperature higher than 150°C, to decompose the polymer into its constituent monomers;
(b) continuously removing the methanol vapours and monomers obtained in step (a) and entrapped by said methanol vapours;
(c) condensing the monomers entrapped by the methanol;
(d) separately condensing and recycling the methanol to step (a);
(e) removing the glycols from the mixture of condensed monomers by distillation;
(f) subjecting the residual product of the distillation to hydrolysis; and
(g) recovering the terephthalic acid obtained by crystallization.

The process of the present invention enables "fibre grade" terephthalic acid to be obtained starting directly from the waste products, by-products and used products based on or containing polyalkylene terephthalate.

The materials subjected to the process of the present invention can be waste products from the production of moulded articles or products such as fibres, films, bottles, etc. as well as the moulded products or articles themselves after use. Low molecular weight oligomers obtained as by-products during the polycondensation or process for the production of the polymer, can also be used. These materials are subjected to treatment with methanol vapours in the form of pellets, filaments or any other form which can be suitably handled. The materials to be treated can also be previously granulated in an extruder or fed to the chamber of a screw extruder which in turn feeds the extruded product directly to the treatment zone. Alternatively, the materials to be treated can be melted and continuously injected to the reaction zone. It is not necessary for the material subjected to the process of the present invention to be free from contaminating products. In fact, the process of the present invention is equally effective even when the polyester fibres to be treated are mixed with other natural, artificial or synthetic fibres; or when the polyester is metallized, dyed, pigmented or mixed with other polymers. Similarly, the presence of extraneous products such as paper, films, packaging products, etc. does not influence the process of the present invention.

The treatment with methanol vapours is carried out at atmospheric pressure, at a temperature higher than 150°C, and with an excess of methanol so that the weight ratio methanol/waste or used materials is at least 2, and preferably between 3 and 5. It is preferable for the temperature of the vapours to be higher than the critical temperature of the methanol, i.e. higher than 230°C, in order to avoid liquefying the methanol by increase in pressure; temperatures of between 240 and 260°C are particularly preferred.

During this treatment with methanol the polyalkylene terephthalate is depolymerized into its constituent monomers, such as dimethyl terephthalate, monomethyl terephthalate and alkylene glycol, and into oligomeric products having a polymerization degree of between 5 and 20. The monomers are continuously removed from the reactor together with the methanol vapours, whereas the oligomeric products remain in the reactor. The quantity of fed products to be treated and the quantity of monomers removed are regulated so that, under standard conditions, an oligomeric phase with an almost constant composition is present on the bottom of the reactor.

The excess methanol fed to the reactor facilitates the removal of the monomers as it acts as transporter.

It is preferable for the vapours leaving the reactor to contain at least 50 moles of methanol per mole of dimethyl terephthalate and preferably from 150 to 500 moles of methanol per mole of dimethyl terephthalate.

With the treatment with methanol, up to about 90% of the terephthalic units present in the polyalkylene terephthalate are converted to dimethyl terephthalate in an average treatment time with methanol of 1-10 hours.

If desired, the treatment with methanol vapours can be carried out on the polyalkylene terephthalate in solution in one of its organic solvents, or partially degraded.

The methanol vapours containing the monomers of polyalkylene terephthalate are continuously removed from the reaction zone and processed to remove the excess methanol and separate the single components. For this purpose, the methanol stream leaving the reactor is subjected to partial condensation and the methanol vapours obtained are further condensed and recycled to phase (a) of the process.

The condensate thus obtained is distilled and the vapours at the head comprising alkylene glycol and dialkylene glycol are removed. The products at the bottom comprising dimethyl terephthalate, dihydroxyethyl terephthalate, hydroxyethyl terephthalate and dimethyl isophthalate, are subjected to hydrolysis.

The impurities and extraneous materials contained in the starting waste product are periodically discharged from the reactor where they accumulate.

The hydrolysis process is carried out with cold or hot water fed to the reactor in such a proportion that the ratio water/product to be hydrolized is higher than 2, preferably between 5 and 20. The process is carried out at a temperature higher than 150°C, preferably at a temperature of between 200 and 300°C for an average residence time of 10-60 minutes. The heating can be carried out by means of an external heating jacket around the reactor or by coils outside or inside the reactor. According to a preferred embodiment, the hydrolysis water is in the form of high pressure vapour fed to the reactor from below. In this way the stirring, thermal exchange and a better contact between the hydrolysis water and material to be treated, are ensured.

The resulting solution is subjected to flash at a temperature higher than 100°C, for example between 110 and 150°C, and at a pressure corresponding to the partial pressure of the water at said temperature. The terephthalic acid precipitates and is separated by filtration, using a filter or centrifuge, at the same temperature and pressure. The crystals thus obtained are washed in the same filtration device and dried.

The impurities and extraneous materials contained in the starting waste or by-products are discharged with the crystallization mother liquors.

The following illustrative but non-limiting examples provide a better understanding of the present invention and refer to a preferred embodiment of the process of the present invention.

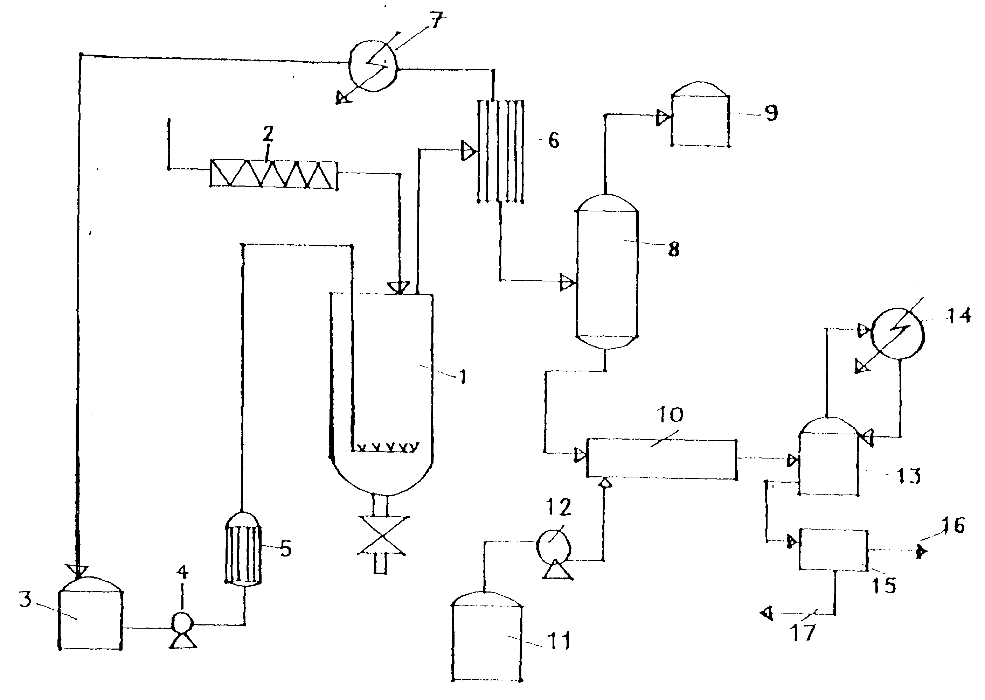

In the examples, the process of the present invention is described with reference to the diagram of the enclosed figure which represents a schematic indicative view of a preferred plant for its embodiment.

In the examples all the parts and percentages are given by weight unless otherwise specified.

### EXAMPLE

The following are continuously fed, under standard conditions, to a reactor (1) having a capacity of 30 l:
- 300 g/h of waste products of polyethylene terephthalate coming from the processing of bottles and fibres, previously granulated by means of a twin-screw extruder (2) and heated to 260°C, and
- 8000 g/h of methanol vapours, removed from a tank (3) by means of a pump (4) and superheated to 255°C by a heat exchanger (5).

The vapours which developed from the reactor (1), were fed to a condenser (6), maintained at a temperature of 140°C, in which the vapours of dimethyl terephthalate, methylhydroxyethyl terephthalate and ethylene glycol were condensed, whereas the methanol vapours were collected and condensed in a second condenser (7) and then fed to the tank (3) in order to be recycled.

After three hours of treatment, the condensed products in the condenser (6) consisted of: 430 g of dimethyl terephthalate, 200 g of methylhydroxyethyl terephthalate and 630 g of ethylene glycol.

The condensation products were continuously fed to a distillation column (8), operating under vacuum at a pressure of 5.1 absolute KPa, to separate the ethylene glycol at the head and the other monomers at the bottom.

The ethylene glycol was collected in a tank (9) and could be re-used as a monomer in the synthesis of polyethylene ester.

The monomers at the bottom were fed at a flow rate of 0.5 l/h to a hydrolysis reactor (10), having a capacity of 2 l and maintained at a temperature of 270°C and a pressure of 8.3 MPa. 3500 g/hr of water removed from the container (11) and preheated to 270°C by an exchanger (12) were continuously and contemporaneously fed to the hydrolysis reactor (10). The monomers at the bottom and water were fed in such a quantity that the average residence time of the reagents in the hydrolysis reactor (10) was 30 minutes. The reaction mass was continuously fed at a flow rate of 4000 g/hr to a flash (13) operating at 128°C and a pressure of about 0.2 MPa. The vapours were recycled after condensation by a condenser (14). Terephthalic acid obtained by hydrolysis precipitated from the solution and the slurry at the bottom was filtered in a centrifuge filter (15); the filtrate was collected in (16) and the aqueous solution of methanol, containing impurities such as isophthalic acid and diethylene glycol, was discharged into (17). The methanol can be recovered from the solution by distillation.

450 g/hr of "fibre grade" terephthalic acid having a purity of 99.9% upon gaschromatographic analysis, were obtained.

## Claims

1. Continuous process for the recovery of terephthalic acid from the waste or used products of polyalkylene terephthalate polymers comprising in subjecting said waste or used products to two consecutive treatments, the first carried out with superheated methanol vapours (methanolysis) and the second with water or water vapour at a high temperature (hydrolysis).

2. Continuous process according to claim 1, comprising in:
(a) treating said polymer waste products with methanol vapours, at atmospheric pressure and at a temperature higher than 150°C, to decompose the polymer into its constituent monomers;
(b) continuously removing the methanol vapours and monomers obtained in step (a) and entrapped by said methanol vapours;
(c) condensing the monomers entrapped by the methanol;
(d) separately condensing and recycling the methanol to step (a);
(e) removing the glycols from the mixture of condensed monomers by distillation;
(f) subjecting the residual product of the distillation to hydrolysis; and
(g) recovering the terephthalic acid obtained by crystallization.

3. Continuous process according to claims 1 or 2, wherein the treatment with methanol vapours is carried out at atmospheric pressure, at a temperature higher than 150°C, and with a weight ratio methanol/waste or used materials of at least 2.

4. Continuous process according to claim 3, wherein the weight ratio methanol/waste or used materials is between 3 and 5.

5. Continuous process according to any of the previous claims, wherein the temperature of the methanol vapours is higher than 230°C, and preferably between 240 and 260°C.

6. Continuous process according to any of the previous claims, wherein the methanol vapours leaving the reactor are subjected to partial condensation, the condensate obtained is subjected to distillation and the vapours at the head are removed and the products at the bottom subjected to hydrolysis.

7. Continuous process according to any of the previous claims, wherein the hydrolysis is carried out with cold or hot water with a ratio water/product to be hydrolized higher than 2, at a temperature higher than 150°C and an average residence time of 10-60 minutes.

8. Continuous process according to claim 7, wherein the ratio water/product to be hydrolized is between 5 and 20 and the temperature is between 200 and 300°C.

9. Continuous process according to any of the previous claims, wherein the solution resulting from the hydrolysis process is subjected to flash at a temperature higher than 100°C, preferably between 110 and 150°C, and a pressure corresponding to the partial pressure of the water and said temperature, and the precipitated terephthalic acid is separated by filtration.

10. Continuous process according to any of the previous claims, wherein the polyalkylene terephthalate polymer is polyethylene terephthalate.

## Patentansprüche

1. Kontinuierliches Verfahren zur Wiedergewinnung von Terephthalsäure aus dem Abfall oder gebrauchten Produkten von Polyalkylenterephthalatpolymeren, umfassend Unterwerfen des Abfalls oder der gebrauchten Produkte unter zwei aufeinanderfolgende Behandlungen, wobei die erste mit überhitzten Methanoldämpfen (Methanollyse) und die zweite mit Wasser oder Wasserdampf bei einer hohen Temperatur (Hydrolyse) durchgeführt wird.

2. Kontinuierliches Verfahren nach Anspruch 1, umfassend:
(a) Behandeln der Polymerabfallprodukte mit Methanoldämpfen bei atmosphärischem Druck und bei einer Temperatur von mehr als 150°C, um das Polymer in die es aufbauenden Monomere zu zerlegen;
(b) kontinuierliches Entfernen der Methanoldämpfe und der in Schritt (a) erhaltenen und in den Methanoldämpfen eingeschlossenen Monomeren;
(c) Kondensieren der in dem Methanol eingeschlossenen Monomere;
(d) separates Kondensieren und Recycling des Methanols in Schritt (a);
(e) Entfernen der Glykole aus dem Gemisch der kondensierten Monomere durch Destillation;
(f) Unterwerfen des restlichen Produktes der Destillation unter eine Hydrolyse; und
(g) Wiedergewinnen der erhaltenen Terephthalsäure durch Kristallisation.

3. Kontinuierliches Verfahren nach Anspruch 1 oder 2, wobei die Behandlung mit Methanoldämpfen bei atmosphärischem Druck, bei einer Temperatur von mehr als 150°C und mit einem Gewichtsverhältnis von Methanol/Abfall oder den gebrauchten verwendeten Materialien von mindestens 2 durchgeführt wird.

4. Kontinuierliches Verfahren nach Anspruch 3, wobei das Gewichtsverhältnis von Methanol/Abfall oder den gebrauchten Materialien zwischen 3 und 5 liegt.

5. Kontinuierliches Verfahren nach einem der vorstehenden Ansprüche, wobei die Temperatur der Methanoldämpfe mehr als 230°C beträgt und vorzugsweise zwischen 240 und 260°C liegt.

6. Kontinuierliches Verfahren nach einem der vorstehenden Ansprüche, wobei die aus dem Reaktor austretenden Methanoldämpfe einer Teilkondensation unterzogen werden, das erhaltene Kondensat einer Destillation unterzogen wird und die Dämpfe am Kopf entfernt werden und die Produkte am Boden einer Hydrolyse unterzogen werden.

7. Kontinuierliches Verfahren nach einem der vorstehenden Ansprüche, wobei die Hydrolyse mit kaltem oder heißem Wasser mit einem Verhältnis von Wasser/zu hydrolisierendem Produkt von mehr als 2, bei einer Temperatur von mehr als 150°C und einer durchschnittlichen Verweilzeit von 10 bis 60 Min. durchgeführt wird.

8. Kontinuierliches Verfahren nach Anspruch 7, wobei das Verhältnis von Wasser/zu hydrolisierendem Produkt zwischen 5 und 20 beträgt und die Temperatur zwischen 200 und 300°C liegt.

9. Kontinuierliches Verfahren nach einem der vorstehenden Ansprüche, wobei die aus dem Hydrolyseverfahren resultierende Lösung einer Flash-Destillation bei einer Temperatur von mehr als 100°C, vorzugsweise zwischen 110 und 150°C und einem Druck unterzogen wird, der dem Partialdruck des Wassers und der Temperatur entspricht, und die ausgefällte Terephthalsäure durch Filtration abgetrennt wird.

10. Kontinuierliches Verfahren nach einem der vorstehenden Ansprüche, wobei das Polyalkylenterephthalat Polyethylenterephthalat ist.

## Revendications

1. Procédé continu pour la récupération de l'acide téréphtalique provenant de déchets ou de produits usagés de polymères poly(téréphtalate d'alcylène) consistant à soumettre lesdits déchets ou produits usagés à deux traitements consécutifs, le premier réalisé avec des vapeurs de méthanol surchauffées (méthanolyse) et le second réalisé avec de l'eau ou de la vapeur d'eau à une température élevée (hydrolyse)

2. Procédé continu selon la revendication 1, consistant:
(a) à traiter lesdits déchets polymères avec les vapeurs de méthanol, sous la pression atmosphérique et à une température supérieure à 150°C, à décomposer le polymère en ses monomères constitutifs;
(b) à éliminer en continu les vapeurs de méthanol et les monomères obtenus dans l'étape (a) et piégés par lesdites vapeurs de méthanol;
(c) à condenser les monomères piégés par le méthanol;
(d) à condenser séparément et à recycler le méthanol vers l'étape (a);
(e) à éliminer les glycols du mélange des monomères condensés par distillation;
(f) à soumettre le produit résiduel de la distillation à une hydrolyse; et
(g) à récupérer l'acide téréphtalique obtenu par cristallisation.

3. Procédé continu selon les revendications 1 ou 2, dans lequel le traitement avec les vapeurs de méthanol est réalisé sous la pression atmosphérique, à une température supérieure à 150°C, et avec un rapport pondéral méthanol/déchets ou matières usagées d'au moins 2.

4. Procédé continu selon la revendication 3, dans lequel le rapport pondéral méthanol/déchets ou matières usagées est compris entre 3 et 5.

5. Procédé continu selon l'une quelconque des revendications précédentes, dans lequel la température des vapeurs de méthanol est supérieure à 230°C, et de préférence comprise entre 240 et 260°C.

6. Procédé continu selon l'une quelconque des revendications précédentes, dans lequel les vapeurs de méthanol quittant le réacteur sont soumises à une condensation partielle, le condensat obtenu est soumis à une distillation et les vapeurs de tête sont éliminées et les produits de fond soumis à une hydrolyse.

7. Procédé continu selon l'une quelconque des revendications précédentes, dans lequel l'hydrolyse est réalisée avec de l'eau froide ou de l'eau chaude avec un rapport eau/produit à hydrolyser supérieur à 2, à une température supérieure à 150°C et pendant une durée moyenne de séjour comprise entre 10 et 60 minutes.

8. Procédé continu selon la revendication 7, dans lequel le rapport eau/produit à hydrolyser est compris entre 5 et 20 et la température est comprise entre 200 et 300°C.

9. Procédé continu selon l'une quelconque des revendications précédentes, dans lequel la solution provenant du procédé d'hydrolyse est soumise à une détente éclair à une température supérieure à 100°C, de préférence entre 110°C et 150°C, et sous une pression correspondant à la pression partielle de l'eau à ladite température, et l'acide téréphtalique précipité est séparé par filtration.

10. Procédé continu selon l'une quelconque des revendications précédentes, dans lequel le poly(téréphtalate d'alkylène) est le poly(téréphtalate d'éthylène).
